# EUROPEAN PATENT SPECIFICATION

(11) **EP 4 076 295 B1**
(45) Date of publication and mention of the grant of the patent: **18.02.2026**
(21) Application number: 19839544.4
(22) Date of filing: 18.12.2019
(51) Int. Cl.: A61F 2/95, A61M 25/00

(54) **FIBER SLACK STORAGE WITHIN A DEPLOYMENT SYSTEM HANDLE**
FASERDURCHHANGAUFBEWAHRUNG IN EINEM HANDGRIFF EINES ENTFALTUNGSSYSTEMS
STOCKAGE DE MOU DE FIBRE DANS UNE POIGNÉE DE SYSTÈME DE DÉPLOIEMENT

(43) Date of publication of application: 26.10.2022
(73) Proprietor: W. L. Gore & Associates, Inc., Newark, DE 19711 (US)
(72) Inventor: BEARD, Matthew, S., Newark, Delaware 19711 (US); SOKEL, Justin, W., Newark, Delaware 19711 (US)
(74) Representative: HGF
(86) International application number: PCT/US2019/067107
(87) International publication number: WO 2021/126183

(56) References cited:
- US-A1- 2004 138 734
- US-A1- 2013 268 048
- US-A1- 2017 273 812
- US-B1- 6 352 561

## Description

### FIELD

The present disclosure relates generally to system line storage apparatuses and, more specifically, to system line storage apparatuses for delayed actuation during medical device deployment and associated methods.

### BACKGROUND

Various medical device delivery systems have multiple actuators including multiple wires, strings, fibers, or other suitable system lines that control operation or deployment of one or more components (sleeve delivery constraint and/or an expandable implantable device, for example). Delivery systems with multiple actuators are utilized, for example, to ensure proper procedural steps and methods are occurring at appropriate times of operation, rather than multiple steps occurring simultaneously or without the proper amount of delay between steps. However, among other issues, systems having multiple actuators may be relatively more complex, require larger handles and/or thicker catheters to accommodate additional system lines, and may have increased risk of malfunction from tangling, knotting, or interference of system lines.

### SUMMARY

The present invention relates to a slack storage system for a system line in a medical device as defined in claim 1 and a method of providing the slack as defined in claim 9. Embodiments of the invention are recited in the dependent claims.

According to an example ("Example 1") of the present disclosure, a slack storage system for a system line in a medical device is disclosed. The slack storage system comprises a plurality of slack pockets arranged in series; and a system line having a first end and a second end with a portion of the system line between the first end and the second end defining a slack loop in each slack pocket, the plurality of slack pockets isolating the plurality of slack loops from each other to define slack in the system line such that when the first end of the system line is tensioned the second end of the system line remains untensioned as the slack in the system line is dispensed by each slack loop straightening from each slack pocket without tangling with another one of the slack loops.

Referring to Example 1, each of the plurality of slack pockets may be sized and shaped such that the slack loops may be formed by pressing the system line into the slack pocket with a finger-shaped element. Each of the slack loops may be of approximately the same length. Each of the plurality of slack pockets may be approximately of the same size and shape. The plurality of slack pockets may each be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

Still referring to Example 1, at least one slack loop may be formed in at least one slack pocket with a finger-shaped element configured to insert one or more of the portions of the system line into the series of slack pockets. At least one additional loop may be formed in at least one additional slack pocket. A plurality of slack loops may be formed simultaneously. The size of the at least one slack loop may be adjusted to modify the amount of slack stored. Assembly verification may be provided by feedback when improper tension is detected during slack loop formation.

According to another example ("Example 2") of the present disclosure, a method for providing slack in a system line for a medical device is disclosed. The method comprises positioning a system line adjacent to a series of slack pockets of a slack storage system; and positioning at least one of a plurality of portions of the system line in at least one of the series of slack pockets of the slack storage system to form at least one slack loop, wherein the slack loop stores slack in the system line between a first end of the system line and a second end of the system line.

Referring to Example 2, at least one slack loop may be formed in the at least one slack pocket with a finger-shaped element configured to insert one or more of the portions of the system line into the series of slack pockets. The method may further comprise the step of forming at least one additional slack loop within at least one additional slack pocket. A plurality of slack loops may be formed simultaneously. The method may further comprise the step of adjusting the size of the at least one slack loop to modify the amount of slack stored. The method may further include the step of providing assembly verification by feedback when improper tension is detected during slack loop formation.

Still referring to Example 2, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to yet another example ("Example 3") of the present disclosure, an apparatus to store slack in a system line is disclosed. The apparatus comprises: a plurality of slack pockets arranged in series; wherein the system line is configured to form a slack loop within each slack pocket, the plurality of slack pockets isolating the slack loops from each other; and wherein the plurality of slack pockets are configured such that when tension is applied to the system line, slack is dispensed by each slack loop straightening from each slack pocket without tangling with another of the slack loops.

Referring to Example 3, at least one slack loop may be formed in the at least one slack pocket with a finger-shaped element configured to insert one or more of the portions of the system line into the series of slack pockets. At least one additional slack loop may be formed within at least one additional slack pocket. A plurality of slack loops may be formed simultaneously. The size of the at least one slack loop may be adjusted to modify the amount of slack stored. Assembly verification may be provided by feedback when improper tension is detected during slack loop formation.

Still referring to Example 3, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to another example ("Example 4") of the present disclosure, a method for providing slack in a system line is disclosed. The method comprises: positioning a system line adjacent to a series of slack pockets of a slack storage system; and positioning at least one of a plurality of portions of the system line in at least one of the series of slack pockets of the slack storage system to form at least one slack loop, wherein the slack loop stores slack in the system line between a first end of the system line and a second end of the system line; wherein the at least one portion of the system line is positioned within the at least one slack pocket with a finger-shaped element.

Referring to Example 4, at least one slack loop may be formed in the at least one slack pocket with a finger-shaped element configured to insert one or more of the portions of the system line into the series of slack pockets. The method may further comprise the step of forming at least one additional slack loop within at least one additional slack pocket. A plurality of slack loops may be formed simultaneously. The method may further comprise the step of adjusting the size of the at least one slack loop to modify the amount of slack stored. The method may further include the step of providing assembly verification by feedback when improper tension is detected during slack loop formation.

Still referring to Example 4, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to yet another example ("Example 5") of the present disclosure, an apparatus to store slack in a system line for a medical device is disclosed, the apparatus comprising: a plurality of slack pockets arranged in series; and a system line forming a slack loop into each slack pocket, the plurality of slack pockets isolating the slack loops from each other; wherein the plurality of slack pockets are configured such that when tension is applied to the system line, slack is dispensed by each slack loop straightening from each slack pocket without tangling with another one of the slack loops.

Referring to Example 5, at least one slack loop may be formed in the at least one slack pocket with a finger-shaped element configured to insert one or more of the portions of the system line into the series of slack pockets. At least one additional slack loop may be formed within at least one additional slack pocket. A plurality of slack loops may be formed simultaneously. The size of the at least one slack loop may be adjusted to modify the amount of slack stored. Assembly verification may be provided by feedback when improper tension is detected during slack loop formation.

Still referring to Example 5, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to another example ("Example 6") of the present disclosure, an apparatus to store slack in a system line is disclosed, the apparatus comprising: a plurality of slack pockets arranged in series; a system line forming a slack loop into each slack pocket, the plurality of slack pockets isolating the slack loops from each other; and wherein the plurality of slack pockets are configured such that when tension is applied to the system line, slack is dispensed by each slack loop straightening from each slack pocket without tangling with another of the slack loops.

Referring to Example 6, at least one slack loop may be formed in the at least one slack pocket with a finger-shaped element configured to insert one or more of the portions of the system line into the series of slack pockets. At least one additional slack loop may be formed within at least one additional slack pocket. A plurality of slack loops may be formed simultaneously. The size of the at least one slack loop may be adjusted to modify the amount of slack stored. Assembly verification may be provided by feedback when improper tension is detected during slack loop formation.

Still referring to Example 6, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to yet another example ("Example 7") of the present disclosure, a method for operating an apparatus to store slack in a system line for a medical device is disclosed. The method comprises: positioning a system line adjacent to a series of slack pockets of a slack storage system; positioning a portion of the system line in each of the series of slack pockets to form a slack loop in each of the series of slack pockets, wherein the slack loops store slack in the system line between a first end of the system line and a second end of the system line; and applying tension to the system line so that each slack loop is released from each slack pocket.

Referring to Example 7, at least one slack loop may be formed in the at least one slack pocket with a finger-shaped element configured to insert one or more of the portions of the system line into the series of slack pockets. The method may further comprise the step of forming at least one additional slack loop within at least one additional slack pocket. A plurality of slack loops may be formed simultaneously. The method may further comprise the step of adjusting the size of the at least one slack loop to modify the amount of slack stored. The method may further include the step of providing assembly verification by feedback when improper tension is detected during slack loop formation.

Still referring to Example 7, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

According to another example ("Example 8") of the present disclosure, a method for operating an apparatus to store slack in a system line is disclosed. The method comprises: positioning a system line adjacent to a series of slack pockets of a slack storage system; positioning a portion of the system line in each of the series of slack pockets to form a slack loop in each of the series of slack pockets, wherein the slack loops store slack in the system line between a first end of the system line and a second end of the system line; and applying tension to the system line so that each slack loop is released from each slack pocket.

Referring to Example 8, at least one slack loop may be formed in the at least one slack pocket with a finger-shaped element configured to insert one or more of the portions of the system line into the series of slack pockets. The method may further comprise the step of forming at least one additional slack loop within at least one additional slack pocket. A plurality of slack loops may be formed simultaneously. The method may further comprise the step of adjusting the size of the at least one slack loop to modify the amount of slack stored. The method may further include the step of providing assembly verification by feedback when improper tension is detected during slack loop formation.

Still referring to Example 8, each of the plurality of slack pockets may be sized and shaped such that the slack loops may be formed by pressing the system line into the slack pocket with a finger-shaped element. Each of the slack loops may be of approximately the same length. Each of the plurality of slack pockets may be of approximately the same size and shape. The plurality of slack pockets may be evenly spaced from one another. Each of the plurality of slack pockets may extend in a common direction. Each of the plurality of slack pockets may include a retainer configured to releasably hold a respective one of the slack loops in the slack pockets into which the slack loop is formed. Where the slack pockets include a retainer, the retainer may include any of an adhesive, a mechanical fastener, a bond, and combinations thereof.

The foregoing Examples are just that and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a schematic view of a medical device deployment system, according to some embodiments.
FIG. 2 is another schematic view of a medical device deployment system, according to some embodiments.
FIG. 3 is a cross-section of a portion of a handle including a system line storage system, according to some embodiments.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale but may be exaggerated to illustrate various aspects of the present disclosure, and in that regard, the drawing figures should not be construed as limiting.

### Definitions and Terminology

With respect terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error or minor adjustments made to optimize performance, for example. In the event it is determined that individuals having ordinary skill in the relevant arts would not readily ascertain values for such reasonably small differences, the terms "about" and "approximately" can be understood to mean plus or minus 10% of the stated value.

Medical devices are frequently used to treat the vasculature of human patients. These treatments or procedures are commonly referred to as intraluminal or endovascular procedures. Such devices often include a sleeve. As used herein, the term "sleeve" refers to a primary, secondary, tertiary, etc., sleeve, sheath, or the like, that covers, surrounds, or is otherwise associated with a medical device. **In** some examples, such "sleeves" constrain a medical device toward a collapsed configuration or outer peripheral dimension suitable for medical delivery of the device to a treatment portion of the vasculature of a patient.

For purposes of the disclosure, the term "constrain" may mean (i) to limit the expansion, either through self-expansion or assistance by a device, of the diameter of a medical device or (ii) to cover or surround but not otherwise restrain a medical device (e.g., for storage or biocompatibility reasons and/or to provide protection to the medical device and/or the vasculature).

As used herein, the term "medical device" or "device" refers to stents, grafts, filters, valves, anchors, occluders, and other devices, including other implantable devices, and also includes all of the foregoing constrained in one or more constraints, or sleeves.

As used herein, the term "line" refers to any type of string, cord, thread, fiber, or wire, and can be comprised of metallic, polymeric, or natural materials, including conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol; and high strength polymer fibers such as ultra-high molecular weight polyethylene fibers or aramid fibers.

This disclosure is not meant to be read in a restrictive manner. For example, the terminology used in the application should be read broadly in the context of the meaning those in the field would attribute such terminology.

### Descriptions of the Disclosed Embodiments

Various examples relate to timed (e.g., delayed) operation of a system line of a delivery system according to a pre-determined operation sequence which may be associated with one or more delivery operations for a medical device. Suitable delivery operations controlled by the system line include removal or actuation of sleeves, delivery constraints, steering functions of the delivery system, and others.

**In** various embodiments, a delivery system includes a sleeve or constraint (e.g., a sleeve, fiber, or other feature) for releasably constraining the medical device (e.g., a vascular implant such as a stent or stent-graft) for delivery to a target site in the body. Such a constraint may have a first state releasably constraining an expandable implant toward a delivery configuration suitable for medical delivery, and a second state in which the medical device is permitted some amount of expansion (e.g., partial or full expansion) from a delivery configuration toward a deployed configuration. The deployment system may include a handle with an actuation mechanism which is configured to actuate the sleeve or constraint through tensioning of an associated system line. Alternatively, or additionally, the delivery system may include an actuation mechanism which is configured to actuate a medical steering operation upon actuation of the actuation mechanism through the system line.

In some examples, the system line operates a sleeve or constraint, such as a sleeve or loop, that releasably constrains an implantable device in a diametrically compacted configuration. In some examples, the constraint includes a sleeve (e.g., made of film material) that extends around or surrounds a medical device, such as an implant (e.g., a stent, stent graft, prosthetic valve, or other endoprosthesis).

In examples where the system line is operatively coupled to a constraint, the constraint may define two states: a first, active state and a second, released state. In the first state, a constraint such as a sleeve may have opposite portions or edges releasably held or sewn together, such as a wire or fiber, to maintain a medical device in the delivery configuration. The wire or fiber may form part of the system line or may be a separate component operatively coupled to the system line. In such embodiments, the constraint can be opened, or otherwise disrupted, by displacing, unstitching, or otherwise disengaging the system line from the constraint to allow expansion of the medical device. Such disengagement may occur through the actuation of the actuation mechanism as described above. Further details of such constraining sleeves can be found, for example, in U.S. Pat. No. 6,352,561 issued to Leopold et al. and U.S. Pat. No. 6,551,350 issued to Thornton et al. Other examples of suitable constraints include a sleeve that is retracted, or pulled proximally, toward a trailing end of the medical device to release the medical device. Still other examples of suitable constraints include fibers or tethers that are looped around the medical device and which can be tensioned and/or released to constrain and unconstrain, or release, the medical device. Still other examples of constraints include knit sleeves that unravel upon tensioning, such as those described in U.S. Pat. No. 6,224,627 to Armstrong et al.

Regardless of the constraint employed, in various examples, tensioning of the system line causes the constraint to release such that an associated medical device self-expands or is able to be expanded according to a desired method of delivery of the medical device.

The timing, or operation sequence for actuation of the system line and concomitant release of the constraint can be controlled by incorporating a slack apparatus to help ensure the deployment step occurs at a given time (e.g., after some predetermined delay) within a sequence of steps. For example, by employing a delay, other actuation or delivery operations (e.g., another sleeve release, a partial deployment, a steering operation) may occur prior to the system line activating an associated component (e.g., constraint). Examples of suitable steering functionality can be found in U.S. Pat. No. 9,375,308 to Norris.

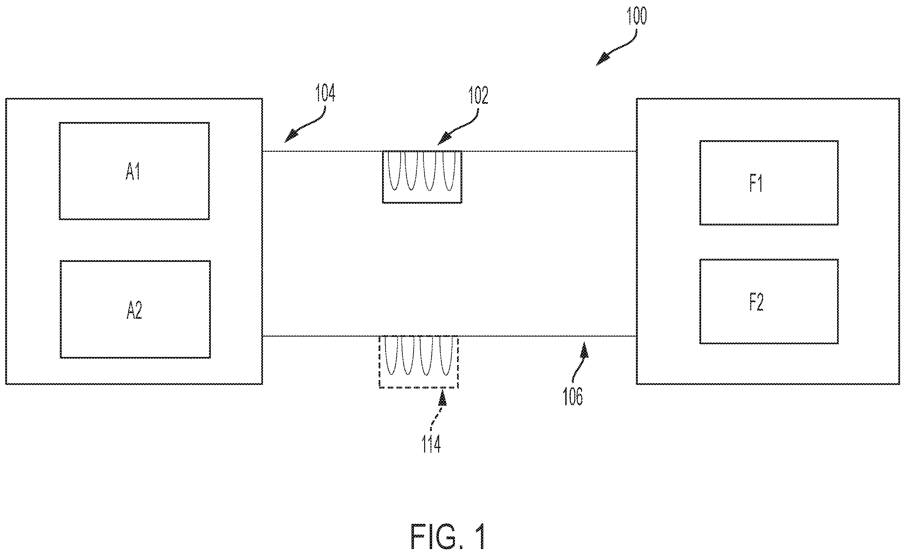

FIG. 1 is a generalized, schematic representation of a medical device deployment system 100, including a system line storage apparatus 102. The medical device deployment system 100 includes a first actuating mechanism A1, a second actuation mechanism A2, a first system line 104, and a second system line 106.

The medical device deployment system 100 may be utilized to carry out a method including a set of operations, or operation elements. In some examples, the deployment system 100 is configured to include at least two operations, or operation elements, a first operation and a second operation, each of which are elements of a delivery operation associated with the system 100. In various examples, the first system line 104 is actuatable via the first actuating mechanism A1 to initiate the first operation element (e.g., actuation of a sleeve or release of a first constraint) and the second system line 106 is actuatable via the second actuating mechanism A2 to initiate the second operation element (e.g., actuation of a sleeve or release of a second constraint).

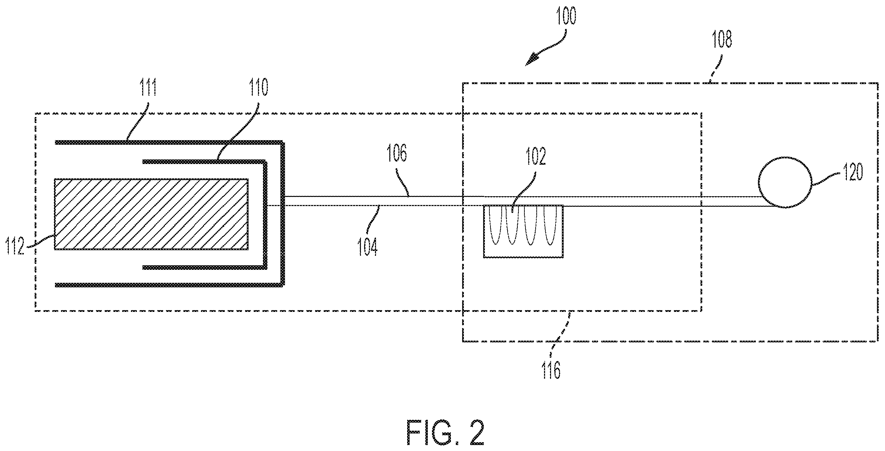

FIG. 2 is another, generalized schematic view of the deployment system 100, according to some examples. As shown in FIG. 2, the first and second actuating mechanisms A1 and A2 may be part of a handle, such as actuation handle 108. The actuation handle 108 may take any of a variety of shapes and sizes and is generally configured for grasping by a user. The actuating mechanisms A1, A2 of FIG. 1 may be embodied in a variety of forms. For example, the actuating mechanisms may be buttons, toggles, switches, dials, rotatable cuffs, or other actuators capable of causing actuation of the first and second system lines 104 and 106, respectively by, for example, giving tension to the first and second system lines 104 and 106, respectively. The first and second actuating mechanisms A1 and A2 may be a single component. For example, the first and second actuating mechanisms A1 and A2 may be part of a single button, toggle, switch, dial, rotatable cuff, or other actuating mechanisms to which both the first and second system lines 104 and 106 are coupled, but which is capable of acting as a first actuation mechanism A1 for the first system line 104 and as a second actuation mechanism A2 for the second system line 106.

Generally, the first actuation mechanism A1 operates to manipulate, for example, apply tension to, the first system line 104, which operates, or actuates the first operation element of the system 100. For example, the first operation element of the system 100 may be the retraction of an outer sleeve 111 (e.g., a first constraining sleeve) overlaying a medical device and maintaining a portion of the medical device at a constrained diameter.

Then, the second actuation mechanism A2 operates to manipulate, for example, apply tension to, the second system line 106 to operate or actuate the second operation element of the system 100. As an example, the second operation element may be the longitudinal displacement of a constraining sleeve (e.g., a second constraining sleeve) over an expandable medical device (e.g., a stent, stent graft, filter, prosthetic valve, occluder, anastomosis device), such as the constraining sleeve 110 and the medical device 112 shown in FIG. 2.

The first system line 104 passes through the system line storage apparatus 102, which holds a desired length or portion of the first system line 104. This length, or stored portion, of the first system line 104 can serve as a timing or delay mechanism such that a desired amount of slack, or stored material, of the system line 104 is tensioned and taken up, or expended, before the system line 104 becomes taught and the second end of the system line 104 beyond the system line storage apparatus 102 actuates, or is tensioned, to cause the first operation element to perform.

In some instances, and as indicated generally in FIG. 1, the system 100 may also include a second system line storage apparatus 114. In such an arrangement, both system lines 104, 106 may be arranged to provide various amounts of delay to the operation elements as desired. In other words, the system line storage apparatus 102 can include a first system line storage apparatus 102, a second system line storage apparatus 114, or multiple system line storage apparatuses depending upon the operation elements and/or the amount of delay desired in the system 100. Although two system lines and one or two storage systems are described and exemplified, it should be clear from the foregoing that any number and combination of system lines and storage systems may be used depending upon the complexity of the delivery system.

As called out in the schematic view of FIG. 2, the delivery system 100 may include catheter body 116, medical device 112, first constraint 110 constraining a first portion of the medical device 112 in a delivery configuration, second constraint 111 constraining a second portion of the medical device 112 in a delivery configuration, actuation mechanism 120 associated with handle 108, first system line 104 configured to assist with carrying out various operation elements of the delivery system 100 (e.g., retracting or releasing the second constraint 111), system line storage apparatus 102 associated with the delivery system 100 (e.g., housed within or otherwise coupled to the catheter body 116 and/or the handle 108).

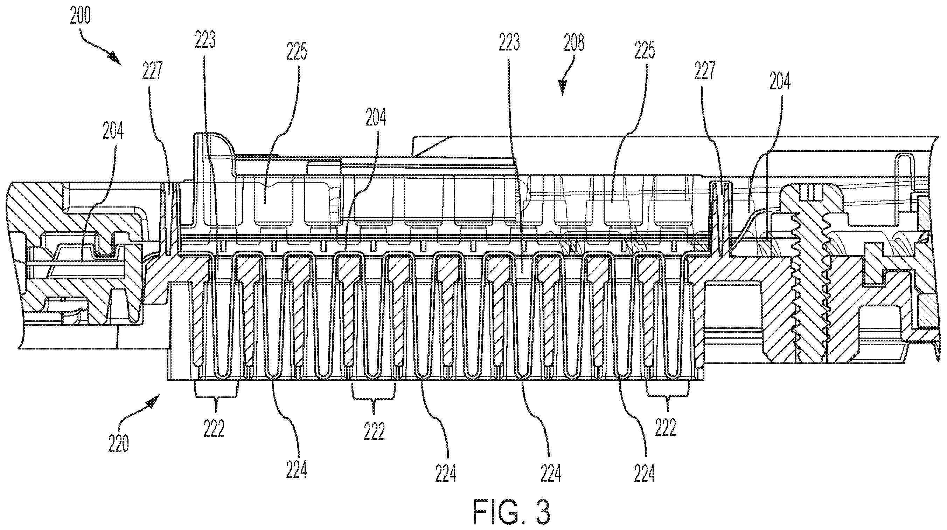

FIG. 3 provides a detailed, sectional view of an example of the handle 108 and the system line storage apparatus 102. For reference, the system line storage apparatus 114 may be similar in shape and form to the storage apparatus 102, as can be any number of system line storage apparatuses included in the delivery system 100.

As shown in the example of FIG. 3, the system line storage apparatus 102 is embodied as apparatus 208 of delivery system 200 (shown housed within a handle of the delivery system 200). The system line storage apparatus 208 includes body 220 defining a plurality of slack pockets 222 arranged in a series within the body 220. Illustratively, the plurality of slack pockets 222 are approximately the same shape and size. In other embodiments, the slack pockets 222 may vary in shape and/or size relative to one another depending on the desire of the user or the requirements of the delivery system 200. For example, in another embodiment of the system line storage apparatus 208 not separately shown, the size and shape of the slack pockets may be altered (e.g., in shape, spacing, orientation, or otherwise) to correspond to particular system needs. As shown, in some examples, the slack pockets 222 each include a neck 223 at the opening of the slack pocket 222, which may vary in width depending on the length of total delay desired and the overall space available within the handle of the delivery system 200.

In general terms, the slack pockets 222 are configured (sized and shaped) to releasably retain the system line 204 (e.g., frictionally whether aided or unaided by secondary retention means such as releasable adhesive or mechanical retainers) until sufficient tension is applied to the system line 204 to retract the system line 204 from the slack pockets 222. The system line storage apparatus may further include one or more retainers configured to hold the slack loops 224 in the slack pockets 222 in which the slack loops 224 are defined. Suitable retainers may include adhesives, mechanical fasteners, and the like and combinations thereof.

Additional retainers may also be utilized to facilitate control of the system line 204. For example, in the exemplary embodiment, the delivery system 200 includes a series of covers 225, each corresponding with a slack pocket 222. Each cover 225 assists with retention of the system line 204 between the slack pockets 222 and includes a thorough hole to allow access to the system line 204 so that the system line 204 may be manipulated to form slack loops 224 within the slack pockets 222. Standing posts 227 may also be positioned on either or both ends of the series of slack pockets 222 to direct the fiber through the delivery system 200. The standing posts 227 facilitate alignment of the fiber with the center of the narrowest portion of the slack pocket when tension is applied to the fiber during assembly, so that the fiber can be consistently pushed down into the slack pockets 222 to a predetermined depth.

In some examples, a pressing element (e.g., a finger-shaped element or simply finger of a user) may be implemented to push, press or otherwise insert the system line 204 into each slack pocket 222 to form a plurality of slack loops 224, wherein each slack loop 224 is formed within in each pocket 222. For example, the slack pockets 222 are sized and shaped to receive the pressing element, which is used to deposit the system line 204 into the slack pockets 222 to hold a given length of the system line 204 as stored slack, or extra length to be taken up during tensioning. For example, as the system line 204 passes through the handle 208, the system line 204 passes over a plurality of openings into the slack pockets 222. A pressing element may be used to press the system line 204 in a direction corresponding to the direction in which the slack pockets 222 extend. As shown, the slack pockets 222 are oriented in a direction substantially perpendicular or orthogonal to the direction in which the system line 204 is to be actuated, or tensioned/pulled. The portions of the system line 204 being engaged by the pressing element are deposited into the slack pockets 222. The pressing element may be removed from the slack pockets 222 once the corresponding portions of the system lines 204 are inserted into the slack pockets 222, leaving the formed slack loops 224 behind in the slack pockets 222.

As alluded to above, the pressing element used to insert the system line 204 into the pockets 222 is moveable so that the slack loops 224 can be formed in a series. For example, the pressing element may be used to insert a first portion of the system line 204 into a first slack pocket 222 to form a first slack loop 224. The pressing element may also be moved to a position above a second portion of the system line 204 lined up with a second slack pocked 222 to insert the second portion of the system line 204 into the second slack pocket 222 to form a second slack loop 224. Such movement can be repeated, with the pressing element moving along to different portions of the system line 204 corresponding to respective slack pockets 222, until each of the slack pockets 222 is full.

The pressing element may comprise a mechanical finger, such as a probe, rod, or other instrument, or, in other embodiments, a user may use his or her finger to insert the system line 204 into the slack pockets 222. In some embodiments, the handle 208 may include multiple pressing elements to form one or more slack loops 224 within the slack pockets 222 simultaneously. For example, in a delivery system 220 having a set number of slack pockets "X", the delivery system may also have "X" number of pressing elements, one for each corresponding slack pocket 222. The delivery system 220 may also have a plurality of pressing elements numbering fewer than the number of slack pockets 222 included in the delivery system 220.

Upon formation of the slack loops 224, each slack loop 224 may be individually or collectively adjusted to modify the amount of slack stored within each slack pocket. Verification of the proper amount of slack can be detected through visual inspection or other feedback following attempted insertion of the system line 204 into a given slack pocket 222. For example, if tension above or below a desired level is detected during testing of a slack loop 224, the process may be deemed defective and redone.

When a desired amount of tension is applied to the system line 204, the slack created by the slack loop 224 is released via the slack loop 224 from each slack pocket 222. The tension is applied to the system line 204 in a direction substantially perpendicular or orthogonal to the direction in which the slack pockets 222, and therefore slack loops 224, extend. This results in a nearly straightening of each slack loop 224 as it is released, paid or, or otherwise removed from the corresponding slack pocket 222. As the slack loops 224 are released in series, there is a reduced likelihood that the system line 204 will tangle with itself or other system components ask the slack in the system line 204 is paid out. The chance of the slack loops 224 tangling with each other is also diminished because the slack pockets 222 can be utilized to isolate the slack loops 224 from each other, meaning that each slack loop 224 is released and straightened in a series, preventing a released slack loop 224 from coming into contact with another slack loop 224 released prior to or after the slack loop being released. The slack pockets 222 may extend in a common direction and/or be evenly spaced from one another to facilitate such a release without tangling of the slack loops 224.

In some embodiments, the slack pockets 222 may extend in alternating directions or have different arrangements depending on the needs of the user and the design of the delivery system without defeating their functionality. Similarly, in some embodiments, the slack pockets 222 may not be evenly spaced depending on the needs of the user and the design of the delivery system. The slack pockets 222 may be disposed in a linear arrangement (e.g., along a common line) as shown, or may be arranged in other configurations, such as an alternating, zig-zag pattern or other arrangement as desired. As referenced, the number and depth of slack pockets 222 may vary depending on the needs of the user and the design of the delivery system. For example, the number of the slack pockets 222, the depth of the slack pockets 222, and the size of the overall assembly may vary to accommodate longer system lines 204 and/or greater amounts of slack built into the system lines 204.

While the illustrative embodiment comprises a system line for use in an implantable medical device, in other embodiments, the slack storage system 220 may be utilized with other system lines associated with other medical devices, such as a suturing system in which the system line is a suture, or in any application where storage of a length of a system line is needed in a manner that avoids tangling as the slack in the system line is paid out. In other words, the invention of this application has been described above both generically and with regard to specific embodiments. It will be apparent to those skilled in the art that various modifications and variations can be made in the embodiments without departing from the scope of the disclosure. The invention is disclosed by the appended claims.

## Claims

1. A slack storage system (220) for a system line (102) in a medical device (100), the slack storage system (220) comprising:
a plurality of slack pockets (222) arranged in series; and
a system line (104, 204) having a first end and a second end with a portion of the system line (104, 204) between the first end and the second end defining a slack loop (224) in each slack pocket (222), the plurality of slack pockets (222) isolating the plurality of slack loops (224) from each other to define slack in the system line (104, 204) such that when the first end of the system line (104) is tensioned the second end of the system line (104, 204) remains untensioned as the slack in the system line (104) is dispensed by each slack loop (224) straightening from each slack pocket (222) without tangling with another one of the slack loops (224).

2. The apparatus of claim 1, wherein each of the plurality of slack pockets (222) are sized and shaped such that the slack loops (224) may be formed by pressing the system line (104, 204) into the slack pocket (222) with a finger-shaped element.

3. The apparatus of claims 1 or 2, wherein each of the slack loops (224) are of approximately the same length.

4. The apparatus of any preceding claim, wherein each of the plurality of slack pockets (222) are of approximately the same size and shape.

5. The apparatus of any preceding claim, wherein the plurality of slack pockets (222) are each evenly spaced from one another.

6. The apparatus of any preceding claim, wherein each of the plurality of slack pockets (222) extend in a common direction.

7. The apparatus of any preceding claim, wherein each of the plurality of slack pockets (222) includes a retainer configured to releasably hold a respective one of the slack loops (224) in the slack pockets (222) into which the slack loop (224) is formed.

8. The apparatus of claim 7, wherein the retainer includes any of an adhesive, a mechanical fastener, a bond, and combinations thereof.

9. A method for providing slack in a system line (104, 204) for a medical device (100), the method comprising:
positioning a system line (104, 204) adjacent to a series of slack pockets (222) of a slack storage system (220); and
positioning at least one of a plurality of portions of the system line (104, 204) in at least one of the series of slack pockets (222) of the slack storage system (220) to form at least one slack loops (224), the at least one of the series of slack pockets (222) isolating the at least one slack loop (224) from the remaining slack pockets (222), wherein the slack loop (224) stores slack in the system line (104, 204) between a first end of the system line and a second end of the system line.

10. The method of claim 9, wherein the at least one slack loops (224) is formed in the at least one slack pocket (222) with a finger-shaped element configured to insert one or more of the portions of the system line (104, 204) into the series of slack pockets (222).

11. The method of claim 9, further comprising the step of forming at least one additional slack loop (224) within at least one additional slack pocket (222).

12. The method of claim 9, wherein a plurality of slack loops (224) are formed simultaneously.

13. The method of any of claims 9-12, the method further comprising the step of adjusting the size of the at least one slack loop (224) to modify the amount of slack stored.

14. The method of any of claims 9-13, the method further including the step of providing assembly verification by feedback when improper tension is detected during slack loop (224) formation.

## Patentansprüche

1. Durchhangaufbewahrungssystem (220) für eine Systemleitung (102) in einer medizinischen Vorrichtung (100), das Durchhangaufbewahrungssystem (220) umfassend:
eine Vielzahl von Durchhangtaschen (222), die in Reihe angeordnet sind; und
eine Systemleitung (104, 204), die ein erstes Ende und ein zweites Ende aufweist, wobei ein Abschnitt der Systemleitung (104, 204) zwischen dem ersten Ende und dem zweiten Ende eine Durchhangschleife (224) in jeder Durchhangtasche (222) definiert, die Vielzahl von Durchhangtaschen (222) die Vielzahl von Durchhangschleifen (224) voneinander isoliert, um einen Durchhang in der Systemleitung (104, 204) zu definieren sodass, wenn das erste Ende der Systemleitung (104) gespannt ist, das zweite Ende der Systemleitung (104, 204) ungespannt bleibt, da der Durchhang in der Systemleitung (104) durch jede Durchhangschleife (224), die sich aus jeder Durchhangtasche (222) gerade richtet, ohne sich mit einer anderen Durchhangschleife (224) zu verwickeln, ausgegeben wird.

2. Gerät nach Anspruch 1, wobei jede der Vielzahl von Durchhangtaschen (222) bemessen und geformt ist, sodass die Durchhangschleifen (224) durch Drücken der Systemleitung (104, 204) in die Durchhangtasche (222) mit einem fingerförmigen Element gebildet werden können.

3. Gerät nach Anspruch 1 oder 2, wobei jede der Durchhangschleifen (224) von ungefähr der gleichen Länge ist.

4. Gerät nach einem vorhergehenden Anspruch, wobei jede der Vielzahl von Durchhangtaschen (222) von ungefähr der gleichen Größe und Form ist.

5. Gerät nach einem vorhergehenden Anspruch, wobei die Vielzahl von Durchhangtaschen (222) jeweils gleichmäßig voneinander beabstandet ist.

6. Gerät nach einem vorhergehenden Anspruch, wobei sich jede der Vielzahl von Durchhangtaschen (222) in eine gemeinsame Richtung erstreckt.

7. Gerät nach einem vorhergehenden Anspruch, wobei jede der Vielzahl von Durchhangtaschen (222) einen Halter beinhaltet, der konfiguriert ist, um eine jeweilige der Durchhangschleifen (224) lösbar in den Durchhangtaschen (222) zu halten, in denen die Durchhangschleife (224) gebildet ist.

8. Gerät nach Anspruch 7, wobei die Halterung ein beliebiges von einem Klebstoff, einem mechanischen Befestigungselement, einer Bindung und Kombinationen davon beinhaltet.

9. Verfahren zum Bereitstellen eines Durchhangs in einer Systemleitung (104, 204) für eine medizinische Vorrichtung (100), das Verfahren umfassend:
Positionieren einer Systemleitung (104, 204) angrenzend an eine Reihe von Durchhangtaschen (222) eines Durchhangaufbewahrungssystems (220); und
Positionieren von mindestens einem einer Vielzahl von Abschnitten der Systemleitung (104, 204) in mindestens einer der Reihe von Durchhangtaschen (222) des Durchhangaufbewahrungssystems (220), um mindestens eine Durchhangschleife (224) zu bilden, wobei die mindestens eine der Reihe von Durchhangtaschen (222) die mindestens eine Durchhangschleife (224) von den übrigen Durchhangtaschen (222) isoliert, wobei die Durchhangschleife (224) den Durchhang in der Systemleitung (104, 204) zwischen einem ersten Ende der Systemleitung und einem zweiten Ende der Systemleitung aufbewahrt.

10. Verfahren nach Anspruch 9, wobei die mindestens eine Durchhangschleife (224) in der mindestens einen Durchhangtasche (222) mit einem fingerförmigen Element gebildet ist, das konfiguriert ist, um einen oder mehrere der Abschnitte der Systemleitung (104, 204) in die Reihe von Durchhangtaschen (222) einzuführen.

11. Verfahren nach Anspruch 9, ferner umfassend den Schritt eines Bildens mindestens einer zusätzlichen Durchhangschleife (224) in mindestens einer zusätzlichen Durchhangtasche (222).

12. Verfahren nach Anspruch 9, wobei eine Vielzahl von Durchhangschleifen (224) gleichzeitig gebildet wird.

13. Verfahren nach einem der Ansprüche 9-12, das Verfahren ferner umfassend den Schritt eines Einstellens der Größe der mindestens einen Durchhangschleife (224), um die Menge an aufbewahrtem Durchhang zu modifizieren.

14. Verfahren nach einem der Ansprüche 9-13, wobei das Verfahren ferner den Schritt eines Bereitstellens einer Anordnungsüberprüfung durch Rückmeldung beinhaltet, wenn während Bilden der Durchhangschleife (224) eine unangemessene Spannung erkannt wird.

## Revendications

1. Système de stockage de mou (220) pour une ligne de système (102) dans un dispositif médical (100), le système de stockage de mou (220) comprenant :
une pluralité de poches de mou (222) agencées en série ; et
une ligne de système (104, 204) possédant une première extrémité et une seconde extrémité, une partie de la ligne de système (104, 204) entre la première extrémité et la seconde extrémité définissant une boucle de mou (224) dans chaque poche de mou (222), la pluralité de poches de mou (222) isolant la pluralité de boucles de mou (224) les unes des autres de manière à définir le mou dans la ligne de système (104, 204) de sorte que lorsque la première extrémité de la ligne de système (104) est mise en tension, la seconde extrémité de la ligne de système (104, 204) reste relâchée lorsque le mou dans la ligne de système (104) est distribué par chaque boucle de mou (224) se redressant de chaque poche de mou (222) sans s'emmêler avec une autre des boucles de mou (224).

2. Appareil selon la revendication 1, chacune de la pluralité de poches de mou (222) étant dimensionnée et profilée de sorte que les boucles de mou (224) puissent se former en enfonçant la ligne de système (104, 204) dans la poche de mou (222) avec un élément en forme de doigt.

3. Appareil selon la revendication 1 ou 2, chacune des boucles de mou (224) présentant approximativement la même longueur.

4. Appareil selon l'une quelconque des revendications précédentes, chacune de la pluralité de poches de mou (222) étant approximativement de la même taille et de la même forme.

5. Appareil selon l'une quelconque des revendications précédentes, ladite pluralité de poches de mou (222) étant chacune espacées uniformément les unes des autres.

6. Appareil selon l'une quelconque des revendications précédentes, chacune de la pluralité de poches de mou (222) s'étendant suivant une direction commune.

7. Appareil selon l'une quelconque des revendications précédentes, chacune de la pluralité de poches de mou (222) comprenant un dispositif de retenue conçu pour maintenir de manière libérable l'une respective des boucles de mou (224) dans les poches de mou (222) dans lesquelles la boucle de mou (224) est formée.

8. Appareil selon la revendication 7, ledit dispositif de retenue comprenant un quelconque élément parmi un adhésif, un dispositif de fixation mécanique, une liaison et des combinaisons de ceux-ci.

9. Procédé permettant de fournir un mou dans une ligne de système (104, 204) pour un dispositif médical (100), le procédé comprenant :
le positionnement d'une ligne de système (104, 204) adjacente à une série de poches de mou (222) d'un système de stockage de mou (220) ; et
le positionnement d'au moins l'une d'une pluralité de parties de la ligne de système (104, 204) dans au moins l'une de la série de poches de mou (222) du système de stockage de mou (220) de manière à former au moins une boucle de mou (224), ladite au moins une de la série de poches de mou (222) isolant ladite au moins une boucle de mou (224) des poches de mou restantes (222), ladite boucle de mou (224) stockant le mou dans la ligne de système (104, 204) entre une première extrémité de la ligne de système et une seconde extrémité de la ligne de système.

10. Procédé selon la revendication 9, ladite au moins une boucle de mou (224) étant formée dans ladite au moins une poche de mou (222) avec un élément en forme de doigt conçu pour insérer une ou plusieurs des parties de la ligne de système (104, 204) dans la série de poches de mou (222).

11. Procédé selon la revendication 9, comprenant en outre l'étape de formation d'au moins une boucle de mou supplémentaire (224) à l'intérieur d'au moins une poche de mou supplémentaire (222).

12. Procédé selon la revendication 9, une pluralité de boucles de mou (224) étant formées en même temps.

13. Procédé selon l'une quelconque des revendications 9 à 12, le procédé comprenant en outre l'étape d'ajustement de la taille de ladite au moins une boucle de mou (224) de manière à modifier la quantité de mou stockée.

14. Procédé selon l'une quelconque des revendications 9 à 13, le procédé comprenant en outre l'étape de fourniture d'une vérification d'assemblage par rétroaction lorsqu'une tension incorrecte est détectée pendant la formation de la boucle de mou (224).
